# EUROPEAN PATENT APPLICATION

(11) **EP 1 806 116 A1**
(43) Date of publication of application: **11.07.2007**
(21) Application number: 06125849.7
(22) Date of filing: 11.12.2006
(51) Int. Cl.: A61F 9/008

(54) **Illumination characteristic selection system for imaging during an ophthalmic laser procedure and associated methods**

(30) Priority: 22.12.2005 US 316200
(71) Applicant: Alcon RefractiveHorizons, Inc., Fort Worth, Texas 76134-2099 (US)
(72) Inventor: LeBlanc, Richard Alan, Clermont, FL 34715-7706 (US); Campin, John A., Orlando, FL 32828 (US); Nguyen, Phuock K., Winter Springs, FL 32708 (US)
(74) Representative: Hanna, Peter William Derek

(57) **Abstract**

A system (10,10') for optimizing the effects of light sources on viewing and image processing, and for imaging an illuminated object (14) takes advantage of the fact that specularly reflected light has particular polarization properties compared with diffusely scattered light. The system includes a polarized light source (11, 20) to illuminate an object with a polarized beam (19) and an unpolarized light source (12) for delivering unpolarized light to illuminate the object with an unpolarized beam (23). A detector (18) is positioned to receive light from the beams reflected from the object. A polarizer (21) is oriented for substantially filtering out specular reflection from the object and for permitting at least a portion of diffuse reflection from the object to pass therethrough. The polarized and unpolarized light sources (11, 12) and the polarizer (21) are controllable for selectively illuminating the object with and detecting light having a selected polarization characteristic as a function of a portion of a procedure being performed.

## Description

### Field of the Invention

The present invention is directed to a system and method for performing a laser ophthalmic surgical procedure, and, more particularly, to a system and method for adjusting an illumination characteristic during the surgical procedure.

### Background of the Invention

Reflections can have a negative impact on image processing, especially if reflections mask an important part of the image. For example, in ophthalmic devices it is sometimes necessary to detect and locate features of an eye such as a pupil. One or more illumination sources are typically necessary in order to achieve sufficient image brightness. These sources can be visible in the image of the eye and can cause problems in locating the desired features. For example, a laser refractive surgery system can have an illumination arrangement that places the sources near the optical axis of the camera. This would cause reflections from the cornea close to the pupil boundary, so that this boundary would be difficult to detect. The problem is exacerbated if a corneal flap is cut on the eye, as the size of the illumination source reflection grows owing to the roughening of the corneal surface. However, if the illumination source is far off-axis, accessibility to the patient is reduced, as is the user's working area.

As a particular goal of laser surgical systems is to provide maximum patient access, it is believed desirable to place illumination sources within a predetermined volume, wherein reflection can affect the image. However, another goal of refractive surgery is to operate on undilated eyes, wherein the pupil is small. For small-spot refractive surgery systems, stabilizing the eye is critical for best outcomes. Tracking undilated eyes demands a determination of pupil size, in which case the area around the pupil must be free of distractions. For large pupils, some obscuration is permissible, since the large perimeter of the pupil allows accurate sizing, with only a portion being obscured. Small pupils, however, can be obscured, which makes them more difficult to track in the presence of distractions. For the present purposes, the transition from "small" to "large" occurs at approximately 4 mm. Therefore, it is preferable that no reflections occur in the image within 4 mm of the pupil center.

Therefore, it would be desirable to provide a system and method of reducing the impact of reflections on an image, by, for example, reducing the intensity of such reflections, without impacting the user's view during critical portions of the procedure.

### Summary of the Invention

The present invention is directed to a system and method for optimizing the effects of light sources on viewing and image processing, and for imaging an illuminated object, in accordance with claims which follow. The system takes advantage of the fact that specularly reflected light (glint) has particular polarization properties compared with diffusely scattered light. The system can comprise means for delivering polarized light to illuminate an object with a polarized beam and means for delivering unpolarized light to illuminate the object with an unpolarized beam. A detector is positioned to receive light from the polarized and the unpolarized beams reflected from the object. A polarizer is positionable upstream of the detector and downstream of the object, and is oriented for substantially filtering out specular reflection from the object and for permitting at least a portion of diffuse reflection from the object to pass therethrough. Means are provided for controlling the polarized and unpolarized light delivering means and the polarizer for selectively illuminating the object with and detecting light having a selected polarization characteristic as a function of a portion of a procedure being performed.

The features that characterize the invention, both as to organization and method of operation, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

### Brief Description of the Drawing

**FIG. 1** is an exemplary system schematic for one embodiment of the present invention.
**FIG. 2** is an exemplary system schematic for another embodiment of the present invention.

### Detailed Description of the Preferred Embodiments

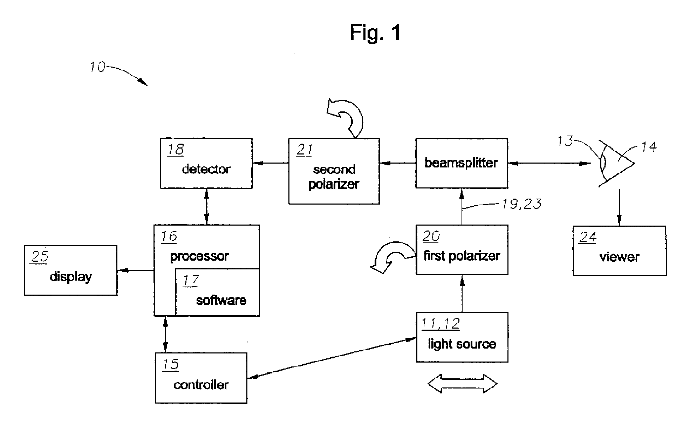

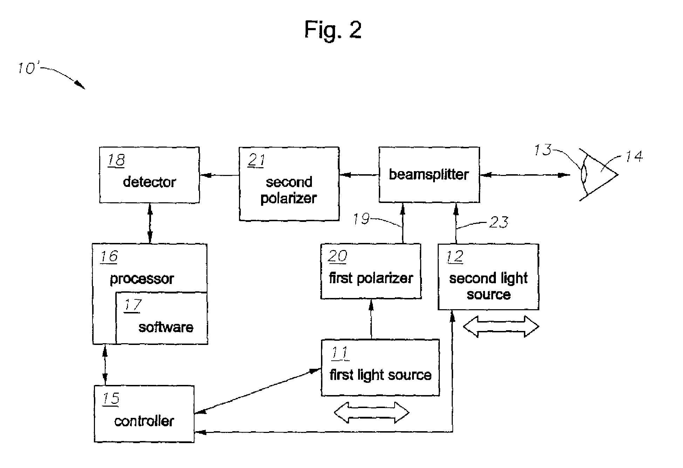

A detailed description of preferred embodiments of the invention will now be discussed with reference to FIGS. 1 and 2. The system **10** of the present invention can comprise a first and a second light source **11, 12** that are positioned to illuminate an object. In the exemplary embodiment, the first **11** and second **12** light source are for illuminating at least a cornea **13** of an eye **14,** and, typically, the area around the cornea **13.** As shown schematically in FIGS. 1 and 2, the system **10** further includes a controller **15** that is in signal communication with the first **11** and second **12** light source. Signal processing means such as a processor **16** with image processing software **17** resident thereon is in communication with a detector **18,** which is positioned to receive light reflected from the eye **14.**

In an embodiment, the first light source **11** is adapted to emit a polarized light beam **19,** for which a first polarizer **20** is positioned downstream of the first light source **11,** and a second polarizer **21** is positioned upstream of the detector **18** and downstream of the eye **14.** The second polarizer **21** is oriented for substantially filtering out specular reflection from the eye **14** and for permitting at least a portion of diffuse reflection from the eye **14** to pass therethrough. The first **20** and the second **21** polarizers in a particular embodiment can be selected from a group consisting of a pair of crossed linear polarizers and a pair of circular polarizers, although these are not intended as limitations.

In an embodiment, the first **21** and the second **22** polarizers are removably positioned for permitting the detection of unpolarized light at the detector **18.** In this system **10,** the first **11** and second **12** light sources can comprise a unitary device (FIG. 1). In another embodiment **10',** the first **11** and the second **12** light sources are separate devices, and the first **21** and the second **22** polarizers can be substantially stationary (FIG. 2).

Either or both of the first **11** and the second **12** light sources may be operated on by the controller **15** to activate them, adjust their brightness, or move them relative to the eye **14.** In addition, the first **11** and the second **12** light sources may be have variable or predetermined spectral composition.

A particular embodiment of a method of the present invention comprises laser refractive surgery on the cornea **13.** Such a procedure is typically carried out in a plurality of stages, wherein different illumination characteristics may be optimal in each stage. For example, if a corneal flap is cut initially to expose underlying stroma, as is known in the art, unpolarized light reflects off the flap and the hinge, causing a "glint" that can be detrimental to eye tracking and image processing systems. However, this glint is useful for locating the flap and hinge.

Thus a hybrid system **10, 10'** can be adapted to provide one or both of polarized **19** and unpolarized **23** beams for different parts of the procedure, and also for different entities receiving the beams **19, 23.** A user viewing the eye **14** through a magnifying device **24** may prefer to see an image illuminated with an unpolarized beam **23** first in order to view the hinge, and then switch to a polarized beam **19** to cut down the glint. Data sent from an image created with an unpolarized beam **23,** however, may be noisy and thus less than optimal for the image processing software **17,** and thus during the procedure it may be preferable to use illumination from a polarized light source **11.** The image processing software **17** can also typically send an image to a display **25** for viewing by the user.

Additional control may further be provided by permitting the light sources **11, 12** to be moved during the procedure in order to optimize a placement of reflections relative to the detector **18.**

Another variable parameter can comprise an ability to adjust other illumination characteristics, such as the wavelength composition of one or both of the beams **19, 23.** Such control would enable, for example, an enhanced viewing of blood vessels with green light during certain portions of the procedure but not during other portions. For example, the blood vessels may be used during alignment of two eye images, but then not be important to see in an enhanced fashion during flap cutting. In another example, if registration marks are made on the eye, the marking ink can have certain predetermined reflection characteristics that can be optimized relative to an illumination beam **19, 23** for improving visualization thereof. In yet a further example, both types of illumination **19, 23** may be used simultaneously, with a brightness of one different from the other in order to, for example, still view the glint but to a lesser extent compared with the polarized reflection.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the system and method illustrated and described herein are by way of example, and the scope of the invention is not limited to the exact details of construction and use.

Having now described the invention, the construction, the operation and use of preferred embodiments thereof, and the advantageous new and useful results obtained thereby, the new and useful constructions, and reasonable mechanical equivalents thereof obvious to those skilled in the art, are set forth in the appended claims.

## Claims

1. A system (10,10') for illuminating an object to be surgically treated comprising:
means (11,20) for delivering polarized light to illuminate an object with a polarized beam (19);
means (12) for delivering unpolarized light to illuminate the object with an unpolarized beam (23);
a detector (18) positioned to receive light from the polarized and the unpolarized beams reflected from the object (14);
a polarizer (21) positionable upstream of the detector and downstream of the object, oriented for substantially filtering out specular reflection from the object and for permitting at least a portion of diffuse reflection from the object to pass therethrough; and
means (15) for controlling the polarized and unpolarized light delivering means and the polarizer for selectively illuminating the object with and detecting light having a selected polarization characteristic as a function of a portion of a procedure being performed.

2. The system recited in Claim 1, wherein the means for delivering polarized light (20) and the polarizer (21) are selected from a group consisting of a pair (20,21) of crossed linear polarizers and a pair of circular polarizers.

3. The system recited in Claim 1, wherein the controlling means (15) comprises means for preventing the reflected polarized beam (19) from reaching the detector (18), and wherein the polarizer is removably positioned, for permitting the detection of unpolarized light at the detector.

4. The system recited in Claim 5, further comprising signal processing means (16,17) in communication with the detector (18) for receiving detected beam data therefrom and in signal communication with the controlling means (15) for controlling at least one of the polarized and the unpolarized light delivering means for retaining the respective polarized and unpolarized beam (19,23) in a desired position relative to the object (14).

5. The system recited in Claim 1, wherein at least one of the polarized and unpolarized light delivering means comprises means for selecting a spectral composition of the respective polarized and unpolarized beam (19,23), for selectively enhancing an imaging of a portion of the object (14) having a predetermined spectral composition.

6. The system recited in Claim 1, wherein at least one of the polarized and unpolarized light delivering means comprises means for adjusting a brightness of the respective polarized and unpolarized beam (19,23).

7. The system recited in Claim 1, wherein at least one of the polarized and unpolarized light delivering means comprises means for adjusting a positioning of the respective polarized and unpolarized beam (19,23).

8. The system recited in Claim 1, further comprising a processor (16) in signal communication with the detector (18) having image processing software (17) resident thereon.

9. The system recited in Claim 8, further comprising a magnifying device positioned to transmit a real-time image of the illuminated object (14) to a user, and wherein the controlling means (15) comprises a device under control of the user.

10. The system recited in Claim 8, further comprising a display device in signal communication with the processor (16) for displaying to a user an image processed by the image processing software (17).

11. The system recited in Claim 8, wherein the controlling means (15) comprises a software routine resident on the processor (16) adapted to receive input indicative of the procedure portion being performed, to determine an illumination characteristic optimal for the procedure portion, and to direct the means for delivering polarized and unpolarized light to achieve the optimal illumination characteristic.

12. A method for illuminating an object (14) to be surgically treated comprising the steps of:
delivering polarized light to illuminate an object with a polarized beam (19);
delivering unpolarized light to illuminate the object with an unpolarized beam (23);
detecting light from the polarized and the unpolarized beams reflected from the object;
if desired, polarizing the detected light for substantially filtering out specular reflection from the object and for permitting at least a portion of diffuse reflection from the object to pass therethrough; and
controlling the polarized and unpolarized beams and the polarizing step for selectively illuminating the object with and detecting light having a selected polarization characteristic as a function of a portion of a procedure being performed.

13. The method recited in Claim 12, wherein the polarized light delivering and polarizing steps are performed with the use of a pair of polarizers (20,21) selected from a group consisting of a pair of crossed linear polarizers and a pair of circular polarizers.

14. The method recited in Claim 13, wherein the controlling step comprises removing the pair of polarizers (20,21) for permitting the detection of unpolarized light at a detector (18).

15. The method recited in Claim 12, further comprising the steps of receiving detected beam data from the detected light and controlling at least one of the polarized and the unpolarized light beams (19,23) for retaining the respective polarized and unpolarized beam in a desired position relative to the object (14).

16. The method recited in Claim 12, wherein at least one of the polarized and unpolarized light delivering steps comprises selecting a spectral composition of the respective polarized and unpolarized beam (19,23), for selectively enhancing an imaging of a portion of the object (14) having a predetermined spectral composition.

17. The method recited in Claim 12, wherein at least one of the polarized and unpolarized light delivering steps comprises adjusting a brightness of the respective polarized and unpolarized beam (19,23).

18. The method recited in Claim 12, wherein at least one of the polarized and unpolarized light delivering steps comprises adjusting a positioning of the respective polarized and unpolarized beam (19,23).

19. The method recited in Claim 12, further comprising transmitting data from the light detecting step to image processing software (17), processing the data, and using the data to automatically determine the polarization characteristic for the controlling step.

20. The method recited in Claim 19, further comprising the step of transmitting a real-time image of the illuminated object (14) to a user, and wherein the controlling step is performed at least in part by the user.

21. The method recited in Claim 19, further comprising the step of displaying to a user an image processed by the image processing software (17).

22. The method recited in Claim 19, wherein the controlling step comprises receiving into a software routine input indicative of the procedure portion being performed, automatically determining an illumination characteristic optimal for the procedure portion, and directing the polarized and unpolarized light delivering steps to achieve the optimal illumination characteristic.
